# EUROPÄISCHE PATENTANMELDUNG

(11) **EP 0 806 192 A2**
(43) Veröffentlichungstag der Anmeldung: **12.11.1997**
(21) Anmeldenummer: 97103904.5
(22) Anmeldetag: 08.03.1997
(51) Int. Cl.: A61F 2/30, A61F 2/36, A61L 27/00

(54) **Halbzeug zur passgenauen Herstellung einer zu implantierenden Prothese**

(30) Priorität: 11.05.1996 DE 19619166
(71) Anmelder: Heraeus Kulzer GmbH, 63450 Hanau (DE)
(72) Erfinder: Schödel, Dieter, Dr., 65193 Wiesbaden (DE)
(74) Vertreter: Kühn, Hans-Christian

(57) **Zusammenfassung**

Die Erfindung betrifft ein Halbzeug zur paßgenauen Herstellung einer zu implantierenden Prothese, die vor dem Einsetzen in den menschlichen oder tierischen Körper durch abtragende Bearbeitung den Abmessungen der für den Einsatz bestimmten Knochenhöhle maßgenau angepaßt wird, und das einen Metallkern aufweist. Um Stoßbelastungen zwischen Prothese und Knochen zu dämpfen und einen festen Sitz der Prothese in der Knochenhöhle zu ermöglichen, ist der Metallkern in einen Mantel aus Knochenzement mindestens teilweise derart eingebettet, daß die Abmessungen des Mantels größer sind, als die entsprechenden Wandabstände in der Knochenhöhle. Die Erfindung betrifft des weiteren ein Verfahren zur Herstellung des Halbzeuges sowie dessen Verwendung.

## Beschreibung

Die Erfindung betrifft ein Halbzeug zur paßgenauen Herstellung einer zu implantierenden Prothese, die vor dem Einsetzen in den menschlichen oder tierischen Körper durch abtragende Bearbeitung den Abmessungen der für den Einsatz bestimmten Knochenhöhle maßgenau angepaßt wird, und das einen Metallkern aufweist. Die Erfindung betrifft weiterhin ein Verfahren zur Herstellung sowie die Verwendung des Halbzeuges.

Derartige Halbzeuge sind zur Herstellung von Hülftgelenksendoprothesen seit einigen Jahren bekannt. Herkömmliche Hüftgelenksendoprothesen werden in eine Aushöhlung des Oberschenkelknochens einzementiert. Dazu werden fertige Prothesenteile verwendet, der Knochen muß bearbeitet, in der Regel ausgehöhlt und damit geschwächt werden. Die Prothese wird mit Hilfe von Knochenzement, der in dem Knochen aushärtet, befestigt. Bei dieser Methode wird der Knochen der Prothese angepaßt. Mit Hilfe des eingangs charakterisierten Halbzeuges ist es möglich, die Prothese dem Knochen anzupassen und so eine Schwächung des Knochens zu vermeiden. Bekannt sind gegossene oder geschmiedete metallische Halbzeuge, beispielsweise aus Titan oder Titanlegierungen oder aus Kobalt-Chrom-Molybdän-Legierungen. Diese Halbzeuge werden in Abmessungen gefertigt, die größer sind, als die Knochenhöhle, in die sie eingesetzt werden sollen. Die Knochenhöhle wird beispielsweise mit Hilfe von Computertomographen auf Bruchteile von Millimetern genau vermessen, die gemessenen Daten werden einer rechnergesteuerten Bearbeitungsmaschine zugeleitet, auf der die Prothese paßgenau, d.h. den Abmessungen der Knochenhöhle entsprechend gefräst wird. Diese metallischen Halbzeuge sind sehr hart und dadurch zum einen schwer zu bearbeiten und zum anderen nicht in der Lage, Stöße oder ähnliche Belastungen abzufangen. Sie sitzen paßgenau in dem Knochen und besitzen kein dämpfendes Element, welches Stöße zwischen den unterschiedlichen Materialien Knochen und Metall abfängt. Dies führt dazu, daß die Gefahr der Lockerung des Prothesenschaftes im Knochen besteht, in Folge dessen der weichere Knochen an dem härteren Metall abreibt und dadurch langsam zerstört wird.

Ausgehend von dem beschriebenen Stand der Technik liegt der Erfindung die Aufgabe zugrunde, ein Halbzeug zu schaffen, das die paßgenaue Herstellung einer Prothese ermöglicht, die sowohl fest in der Knochenhöhle sitzt als auch Stoßbelastungen zwischen der Prothese und dem Knochen dämpft. Aufgabe der Erfindung ist es weiterhin, ein Verfahren zur Herstellung des Halbzeuges sowie seine Verwendung anzugeben.

Erfindungsgemäß wird die Aufgabe für das Halbzeug dadurch gelöst, daß der Metallkern in einen Mantel aus Knochenzement mindestens teilweise derart eingebettet ist, daß die äußeren Abmessungen des Mantels größer sind als die entsprechenden Wandabstände in der Knochenhöhle. Dabei werden die dem Fachmann geläufigen Abmessungen von Knochenhöhlen zugrunde gelegt, da den Maßen des Mantels nach oben hin im wesentlichen nur wirtschaftliche Grenzen gesetzt sind. Insbesondere ist es vorteilhaft, daß die Abmessungen des Metallkerns im wesentlichen kleiner sind, als die entsprechenden Wandabstände in der Knochenhöhle, um zu sichern, daß bei der Implantation keine wesentlichen kraftschlüssigen Verbindungen direkt zwischen Knochenhöhle und Metall entstehen, sondern daß dazwischen Stöße dämpfendes Mantelmaterial (Knochenzement) angeordnet ist. Ein derartiges Halbzeug kann serienmäßig, gegebenenfalls in verschiedenen Konfektionsgrößen bereitgestellt werden. Der Fachmann kann so die richtige Größe entsprechend dem konkreten Fall auswählen. Das Halbzeug (der Mantel) wird in der Regel spanabhebend bearbeitet, beispielsweise gefräst. Die Endmaße werden durch Ausmessen der Knochenhöhle mit den dem Fachmann geläufigen Meßmethoden, beispielsweise der Computertomographie, ermittelt und durch entsprechende Bearbeitung auf das Halbzeug übertragen, so daß das Halbzeug nach seiner Bearbeitung paßgerecht in die Knochenhöhle eingesetzt werden kann. Der Knochenverschleiß wird minimiert und eventuelle Nachteile, die bei der herkömmlichen Zementierung von Metallprothesen auftreten, werden vermieden. Der Knochenzement wirkt dämpfend zwischen dem Material des Knochens und dem Metallkern der Prothese, so daß Stöße und andere Druckbelastungen vermindert werden. Dadurch werden sowohl Abrieb an Knochen und Prothese als auch Schmerzen beim Patienten verhindert. Außerdem kann der Knochen in den Zementmantel einwachsen, so daß der sichere Halt gewährleistet ist.

Vorteilhaft ist es, daß der Knochenzement ein Antibiotikum zur Bekämpfung von Infektionen innerhalb der Knochenhöhle enthält.

Zweckmäßig ist es, daß die Abmessungen des Mantels mindestens 4 mm größer sind, als die entsprechenden Wandabstände der Knochenhöhle. Als Knochenzement sind herkömmliche Materialien, wie Polymethylmethacrylat (PMMA), zum Beispiel die als Palacos oder Septopal bekannten Materialien, oder auch andere Kunststoffe, wie Polysulfone, Polyester oder Polyethylene, möglich.

Zweckmäßig kann es sein, daß zwischen dem Metallkern und dem Mantel aus Knochenzement eine Haftvermittlerschicht angeordnet ist, die beispielsweise auf der Basis von Silikat gebildet ist.

In einer vorteilhaften Ausführungsform weist der Metallkern einen Schaft und einen Kopf auf, wobei der Schaft die Form des Schaftes einer Hüftgelenksendoprothese aufweist, der in dem Mantel aus Knochenzement eingebettet ist.

Vorteilhaft ist es auch, wenn zwischen Schaft und Kopf ein Kragen angeordnet ist, der dann als Anschlag- bzw. Auflagefläche am Eingang der Knochenhöhle dienen kann. Es kann auch zweckmäßig sein, daß der Schaft um seinen Umfang herum Stufen aufweist, die beispielsweise umlaufend oder teilweise umlaufend ausgebildet sind. Diese Stufen dienen zum einen einem besseren Haften des Mantels an dem Schaft, zum anderen unterstützen die Stufen einen festen Sitz der Prothese im Knochen.

Die Aufgabe wird für ein Verfahren zur Herstellung eines Halbzeuges dadurch gelöst, daß auf den Metallkern der Mantel aus Knochenzement im Spritzgußverfahren oder in einer Preßform aufgebracht und anschließend ausgehärtet wird. Neben dem Spritzgußverfahren kann beispielsweise ein Zweikomponentenmaterial, bestehend aus Flüssigkeit und Pulver nach dem Mischen in eine Preßform um den Metallkern herum eingepreßt werden. Vorteilhaft ist es, vor dem Aufbringen des Mantels eine Haftvermittlerschicht auf den Metallkern aufzubringen, beispielsweise durch Flammenpyrolyse oder durch Aufstreichen mit einem Pinsel. Dabei können mehrere Schichten der Haftvermittlerschicht aufgetragen werden.

Das Halbzeug kann zur Herstellung einer in eine Knochenhöhle zu implantierende Prothese verwendet werden, wobei die Abmessungen der Knochenhöhle gemessen werden und nach diesen Abmessungen der Mantel aus Knochenzement gefräst wird. Insbesondere kann das Halbzeug zur Herstellung einer Hüftgelenksendoprothese verwendet werden. Der Knochenzement kann keilförmig gefräst oder auf andere geeignete Weise bearbeitet werden, wobei ein Kragen zwischen dem Schaft und dem Kopf des Prothesenteils angeordnet ist, der als Auflagefläche auf dem Eingang der Knochenhöhle dient.

In dem Knochenzement können auch z. B. umlaufende Stufen gefräst werden, die die Prothesenteile in der Knochenhöhle abstützen. Auch dadurch entsteht eine Druckkomponente, die in Längsrichtung der Knochenhöhle gerichtet ist, so daß höchstens ein geringer Teil der zwischen Knochen und Prothesenteil wirkenden Kraft etwa senkrecht zur Längsachse des Knochens gerichtet ist. Dadurch wird eine Spaltung des Knochens verhindert.

Im folgenden wird ein Ausführungsbeispiel der Erfindung anhand einer Zeichnung näher erläutert.

In die Zeichnung zeigt
- Figur 1: die schematische Darstellung eines Halbzeugs zur Herstellung einer Hüftgelenksendoprothese,
- Figur 2: die schematische Darstellung eines Halbzeugs mit gestuftem Kern und
- Figur 3: eine aus dem Halbzeug gefertigte Hüftgelenksendprothese, wobei der Knochen, in den sie einzusetzen ist, andeutungsweise ebenfalls dargestellt ist.

Das Halbzeug besteht aus einem Kern 1 aus Titan oder einer Titanlegierung. Das Halbzeug weist einen Schaft 2 und einen Kopf 3 auf. Der Schaft 2 ist kleiner als übliche Knochenhöhlen, in denen Hüftgelenksendoprothesen eingesetzt werden; er weist jedoch die Form eines Schaftes einer Hüftgelenksendoprothese auf.

Die Oberfläche des Schaftes 2 ist mit einer Schicht 4 eines Haftvermittlers beschichtet, der die Haftung des Knochenzementes des Mantels 5 auf dem Schaft 2 verbessert.

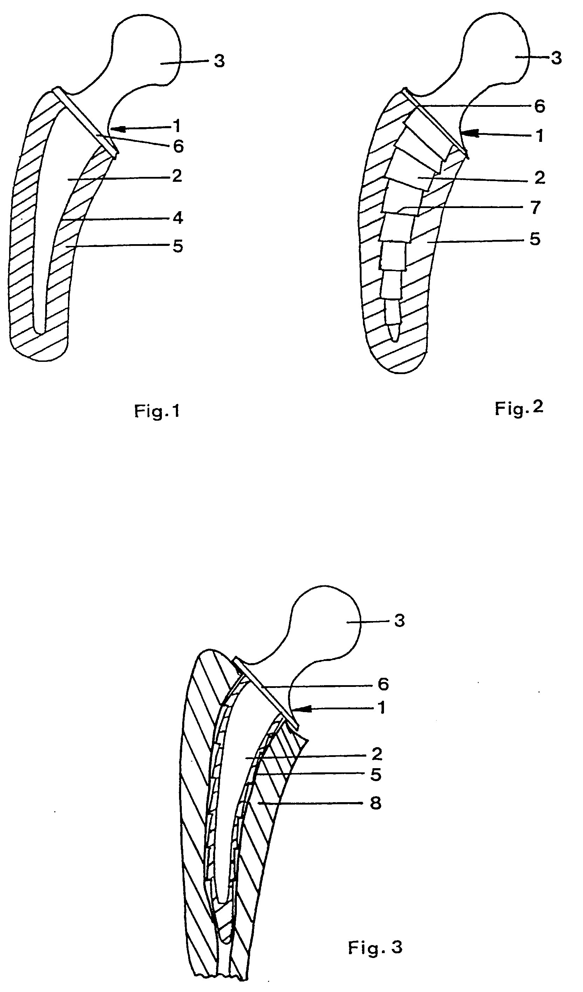

Zwischen Schaft 2 und Kopf 3 ist ein Kragen 6 angeordnet, der als Auflagefläche am Knochen dient. In Figur 2 ist eine Ausführung des Schaftes 2 mit Stufen 7 dargestellt.

Der Haftvermittler kann auf verschiedene Arten aufgebracht werden. Möglich ist das Aufbringen einer glasartigen Silikatschicht mittels bekannter Flammenspritzverfahren. Eine weitere Möglichkeit besteht darin, ein flüssiges Präparat, das Wasser, Aceton, ein Methacrylat und ein reaktives Polymer enthält, beispielsweise mittels eines Pinsels aufzutragen und diesen Überzug durch Erwärmen zu verfestigen. Dabei können zwei Präparate nacheinander auf den Schaft 2 aufgetragen werden, wobei das erste Präparat 5 - 25 Gewichts-% mit einer wäßrigen Copolymersuspension, in der das Copolymer aus einer Mischung von 40 - 80 Gewichts-% Acrylnitril und 60 - 20 Gewichts-% Butylacrylat besteht, 5 - 20 Gewichts-% Wasser, 35 - 75 Gewichts-% polares Lösungsmittel (z.B. Aceton) und 3 - 15 Gewichts-% eines Methacrylates enthält und das zweite Präparat 5 - 20 Gewichts-% einer 40 - 90 gewichts-%igen Lösung eines isocyanatgruppenfreien Polyurethan-Einbrennharzes Insolvent Naphtha 100, 65 - 85 Gewichts-% Lösungsmittel und 3 - 20 Gewichts-% eines Methacrylats enthält. Vor dem Aufbringen der zweiten Schicht wird die erste Schicht zumindest leicht angetrocknet. Nach dem Trocknen der zweiten Schicht wird der Schaft 2 mit dem Mantel 5 aus Knochenzement (beispielsweise Palacos oder Septopal) im Spritzgußverfahren umgeben. Der Mantel 5 kann auch in einer Preßform, in der der Schaft 2 angeordnet wird, aufgebracht werden, indem eine Masse, die beispielsweise aus einer Flüssigkeit und einem Pulver angemischt wurde, hineingepreßt wird.

Der Mantel 5 kann als massiver Block um den Schaft 2 herum angeordnet sein. Bei einer dünneren Ummantelung wird die Dicke des Mantels 5 in der Regel an der Spitze des Schaftes geringer sein als am entgegengesetzten Ende. An der dünnsten Stelle sollte der Mantel eine Stärke von mindestens 2 mm aufweisen. Anonsten ist die Stärke des Mantels 5 nahezu beliebig festsetzbar. Sie muß lediglich groß genug sein, um nach der Bearbeitung den Abmessungen der Knochenhöhle entsprechen zu können.

Die Bearbeitung geschieht dadurch, daß mittels Computertomographie die exakten Abmessungen der Knochenhöhle ermittelt werden. Diese Daten werden auf eine computergesteuerte Fräsmaschine übertragen; dort wird die Prothese exakt den Abmessungen der Knochenhöhle entsprechend fertiggestellt. Der Mantel 5 aus Knochenzement ist dabei sehr leicht zu bearbeiten. Figur 3 zeigt eine gestufte Knochenzementoberfläche, bei der die Stufen an der Wand der Knochenhöhle anliegen, wenn die Prothese in den Knochen 8 implantiert ist.

Dem Knochenzement wird ein Antibiotikum zugesetzt, das über einen längeren Zeitraum freigesetzt wird und verhindert, daß Infektionen in der Knochenhöhle entstehen.

## Patentansprüche

1. Halbzeug zur paßgenauen Herstellung einer zu implantierenden Prothese, die vor dem Einsetzen in den menschlichen oder tierischen Körper durch abtragende Bearbeitung den Abmessungen der für den Einsatz bestimmten Knochenhöhle maßgenau angepaßt wird, und das einen Metallkern aufweist, dadurch gekennzeichnet, daß der Metallkern (1) in einen Mantel (5) aus Knochenzement mindestens teilweise derart eingebettet ist, daß die äußeren Abmessungen des Mantels (5) größer sind, als die entsprechenden Wandabstände in der Knochenhöhle.

2. Halbzeug nach Anspruch 1, dadurch gekennzeichnet, daß die Abmessungen des Metallkerns (1) kleiner sind als die entsprechenden Wandabstände in der Knochenhöhle.

3. Halbzeug nach Anspruch 1 oder 2, dadurch gekennzeichnet, daß der Knochenzement ein Antibiotikum enthält.

4. Halbzeug nach einem der Ansprüche 1 bis 3, dadurch gekennzeichnet, daß die Abmessungen des Mantels (5) mindestens 4 mm größer sind als die entsprechenden Abmessungen der Knochenhöhle.

5. Halbzeug nach einem der Ansprüche 1 bis 4, dadurch gekennzeichnet, daß der Knochenzement ein Polymethylmethacrylat, Polysulfon, Polyester oder Polyethylen ist.

6. Halbzeug nach einem der Ansprüche 1 bis 5, dadurch gekennzeichnet, daß zwischen Metallkern (1) und Mantel (5) eine Haftvermittlerschicht (4) angeordnet ist.

7. Halbzeug nach Anspruch 6, dadurch gekennzeichnet, daß die Haftvermittlerschicht (4) im wesentlichen ein Silikat aufweist.

8. Halbzeug nach einem der Ansprüche 1 bis 7, dadurch gekennzeichnet, daß der Metallkern (1) einen Schaft (2) und einen Kopf (3) aufweist, wobei der Schaft (2) die Form des Schaftes einer Hüftgelenksendoprothese aufweist und in den Mantel (5) aus Knochenzement eingebettet ist.

9. Halbzeug nach Anspruch 8, dadurch gekennzeichnet, daß zwischen Schaft (2) und Kopf (3) ein Kragen (6) angeordnet ist.

10. Halbzeug nach Anspruch 8 oder 9, dadurch gekennzeichnet, daß der Schaft (2) Stufen (7) aufweist, die in Umfangsrichtung des Schaftes (2) verlaufen.

11. Verfahren zur Herstellung eines Halbzeugs nach Anspruch 1, dadurch gekennzeichnet, daß der Mantel (5) im Spritzgußverfahren oder in einer Preßform auf den Metallkern (1) aufgebracht und anschließend ausgehärtet wird.

12. Verfahren nach Anspruch 11, dadurch gekennzeichnet, daß vor dem Aufbringen des Mantels (5) eine Haftvermittlerschicht (4) auf den Metallkern (1) aufgebracht wird.

13. Verfahren nach Anspruch 12, dadurch gekennzeichnet, daß die Haftvermittlerschicht (4) durch Flammenpyrolyse aufgebracht wird.

14. Verwendung eines Halbzeuges nach Anspruch 1 zur Herstellung einer in eine Knochenhöhle zu implantierenden Prothese, wobei die Abmessungen der Knochenhöhle gemessen werden und nach diesen Abmessungen der Mantel (5) aus Knochenzement gefräst wird.

15. Verwendung eines Halbzeuges nach Anspruch 1 zur Herstellung einer Hüftgelenksendoprothese.
